# EUROPEAN PATENT SPECIFICATION

(11) **EP 2 193 747 B1**
(45) Date of publication and mention of the grant of the patent: **06.05.2015**
(21) Application number: 09174827.7
(22) Date of filing: 03.11.2009
(51) Int. Cl.: G01S 15/89, A61B 8/00, A61B 8/08, G01S 7/52

(54) **Ultrasound system and method of providing orientation help view**
Ultraschallsystem und Verfahren zur Bereitstellung einer Ausrichtungshilfsansicht
Système à ultrasons et procédé pour la fourniture d'une vue d'aide à l'orientation

(30) Priority: 02.12.2008 KR 20080121179; 30.01.2009 KR 20090007198
(43) Date of publication of application: 09.06.2010
(73) Proprietor: Samsung Medison Co., Ltd., Hongcheon-gun, Gangwon-do 250-875 (KR)
(72) Inventor: Kim, Yun Jin, 135-851, Seoul (KR); Kim, Sung Hee, 135-851, Seoul (KR)
(74) Representative: Schmid, Wolfgang

(56) References cited:
- EP-A- 1 857 051
- WO-A-97/20288
- US-A1- 2004 138 559
- MERCIER L ET AL: "A review of calibration techniques for freehand 3-D ultrasound systems" ULTRASOUND IN MEDICINE AND BIOLOGY, NEW YORK, NY, US, vol. 31, no. 4, 1 April 2005 (2005-04-01), pages 449-471, XP004849064 ISSN: 0301-5629

## Description

### BACKGROUND OF THE INVENTION

### [Technical Field]

The present invention generally relates to ultrasound systems, and more particularly to an ultrasound system and a method of providing an orientation help (OH) view.

### [Background Art]

The ultrasound system has become an important and popular diagnostic tool due to its non-invasive and non-destructive nature. Modem high-performance ultrasound imaging diagnostic systems and techniques are commonly used to produce two or three-dimensional images of internal features of patients (target objects).

Generally, the ultrasound system may provide a three-dimensional ultrasound image including clinical information such as spatial information and anatomical figures of the target objects, which cannot be provided by a two-dimensional ultrasound image.

Generally, the ultrasound system may provide a three-dimensional ultrasound image including clinical information such as spatial information and anatomical figures of the target objects, which cannot be provided by a two-dimensional ultrasound image.

The ultrasound system may provide an orientation help (OH) function to form an OH view showing a spatial orientation of volume data on a display region. The OH view may be three-dimensionally formed by using entire contours of the ultrasound volume data. The OH view may three-dimensionally indicate a position of reference plane, which may be selected by a user in the volume data, on a three-dimensional space.

In the conventional OH function, only the reference plane may be rotatable. That is, geometrical operations of the OH view such as rotation, reduction, enlargement, movement and the like may not be allowed. Thus, there is a problem since it is difficult for the user to recognize a spatial orientation of the volume data and the reference plane.

EP 1 847 051 A discloses an ultrasonic probe transmitting ultrasonic waves to a subject to be examined and receiving reflected waves from the subject to be examined. An image processor generates three-dimensional image data based on the reflected waves received by the ultrasonic probe. The image processor displays a mark indicating the positional relationship between the three-dimensional image and the ultrasonic probe on a display, the mark overlapping the three-dimensional image based on the three-dimensional image data.

The three-dimensional ultrasound imaging system of WO 97/20288 A includes an ultrasound probe to direct ultrasound waves to and to receive reflected ultrasound waves from a target volume of a subject under examination. The ultrasound probe is swept over the target volume along a linear scanning path and the reflected ultrasound waves are conveyed to a computer wherein successive two-dimensional images of the target volume are digitized. The digitized two-dimensional images can be used to generate a three-dimensional image with virtually no delay. A user interface allows a user to manipulate the displayed image.

US 2004/138599 A1 describes a method and a system for screening and/or diagnosing a tumor in a subject breast using ultrasonically obtained image information. A user interface displays a three-dimensional view of the tumor suspended within a three-dimensional semitransparent view of a breast. A larger, close-up view of the tumor is also provided that a user can manipulate (e.g., rotate, enlarge, etc.) for clearer analysis.

### BRIEF DESCRIPTION OF THE DRAWINGS

FIG. 1 is a bock diagram showing an illustrative embodiment of an ultrasound system.
FIG. 2 is a block diagram showing an ultrasound data acquisition unit.
FIG. 3 is a schematic diagram showing a volume data.
FIG. 4 is a block diagram showing an illustrative embodiment of a processor.
FIG. 5 is a schematic diagram showing an example of an orientation help view.

### DETAILED DESCRIPTION OF THE INVENTION

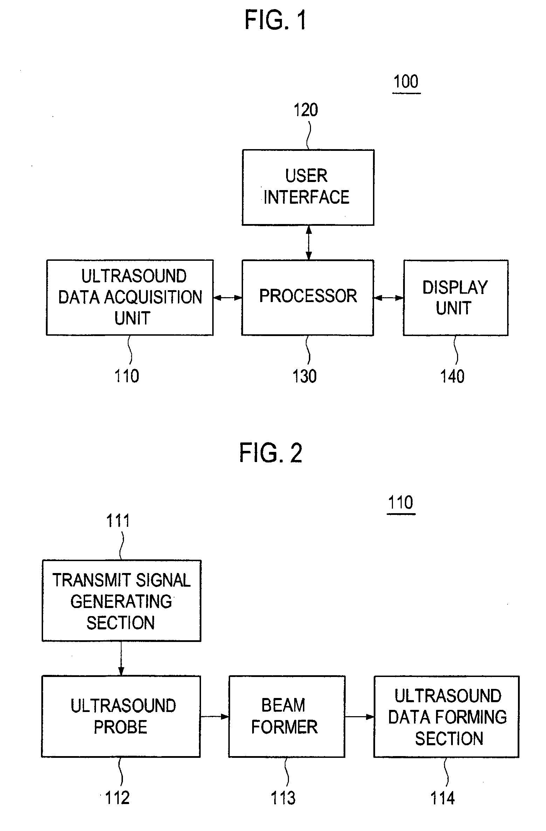

FIG. 1 is a block diagram showing an illustrative embodiment of an ultrasound system. The ultrasound system 100 may include an ultrasound data acquisition unit 110, a user interface 120, a processor 130 and a display unit 140.

The ultrasound data acquisition unit 110 may be configured to transmit ultrasound signals to a target object and receive ultrasound echo signals reflected from the target object. The ultrasound data acquisition unit 110 may be further configured to form ultrasound data of the target object based on the received ultrasound echo signals.

FIG. 2 is a block diagram showing the ultrasound data acquisition unit 110. The ultrasound data acquisition unit 110 may include a transmit (TX) signal generating section 111, an ultrasound probe 112 including a plurality of transducer elements (not shown), a beam former 113 and an ultrasound data forming section 114.

The TX signal generating section 111 may be operable to generate TX signals according to an image mode set in the ultrasound system 100. The image mode may include a brightness (B) mode, a Doppler (D) mode, a color flow mode, etc. In one embodiment, it will be described that the B mode is set in the ultrasound system 100 to obtain a B-mode image.

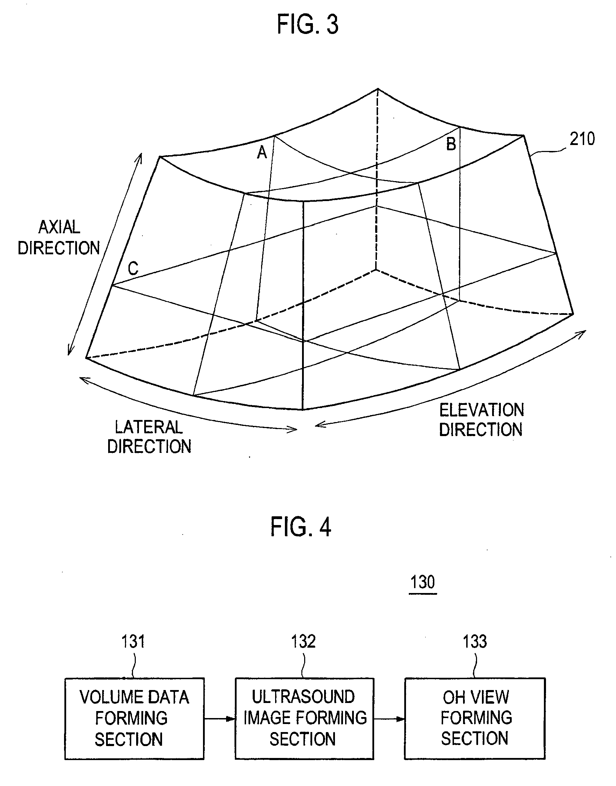

The ultrasound probe 112 may include a three-dimensional probe containing an array transducer having a plurality of transducer elements, which iteratively swing at a prescribed angle along an elevation direction as illustrated in FIG. 3. FIG. 3 is a schematic diagram showing a volume data. The details of FIG. 3 will be explained later.

The ultrasound probe 112 may be operable to transmit the ultrasound signals, which may travel into the target object, along scan lines set in an axial direction responsive to the TX signals received from the TX signal generating section 111. The ultrasound probe 112 may be further operable to receive ultrasound echo signals reflected from the target object and convert them into electrical receive signals. The ultrasound probe 112 may include an orientation detecting section (not shown) for detecting an orientation of the ultrasound probe 112 and a scanning direction of the ultrasound probe 112. In one embodiment, the scanning direction may indicate at least one of a scan line direction and a swing direction of the array transducer.

The beam former 113 may be operable to convert the electrical receive signals outputted from the ultrasound probe 112 into digital signals. The beam former 113 may be further operable to apply delays to the digital signals in consideration of the distances between the transducer elements and focal points to thereby output receive-focused signals.

The ultrasound data forming section 114 may be operable to form an ultrasound data of the target object based on the receive-focused signals. The ultrasound data forming section 114 may be further operable to perform signal processing upon the receive-focused signal such as gain adjustment, filtering and the like.

Referring back to FIG. 1, the user interface 120 may include at least one of a control panel (not shown), a mouse (not shown), a keyboard (not shown) and the like. The user interface 120 may be operable to allow a user to input user instructions. The user instructions may include first, second and third instructions. The first instruction may include setting a region of interest (ROI). The ROI setting may include size and position setting of the ROI. The second instruction may include selecting one of the reference planes. In one embodiment, by way of non-limiting examples, the reference planes may include an A plane, a B plane and a C plane in a volume data 210, as illustrated in FIG. 3. The third instruction may include setting a geometrical operation upon an orientation help (OH) view. The geometrical operation of the OH view may include one of rotation, reduction, enlargement and movement of the OH view.

Referring to FIG. 4, the processor 130 may include a volume data forming section 131, an ultrasound image forming section 132 and an OH view forming section 133. The volume data forming section 131 may be configured to form the volume data based on the ultrasound data. FIG. 3 is a schematic diagram showing a volume data. In FIG. 3, the elevation direction may be the swing direction of the array transducer, the axial direction may be the scan line direction of the array transducer and the lateral direction may be the longitudinal direction of the array transducer.

The ultrasound image forming section 132 may be configured to form the ultrasound images based on the volume data. The ultrasound image forming section 132 may form a three-dimensional ultrasound images as well as images of the reference planes corresponding to the A plane, the B plane and the C plane using the volume data 210 as illustrated in FIG. 3. The OH view forming section 133 may be configured to form an OH view 300 in response to the first and second user instructions.

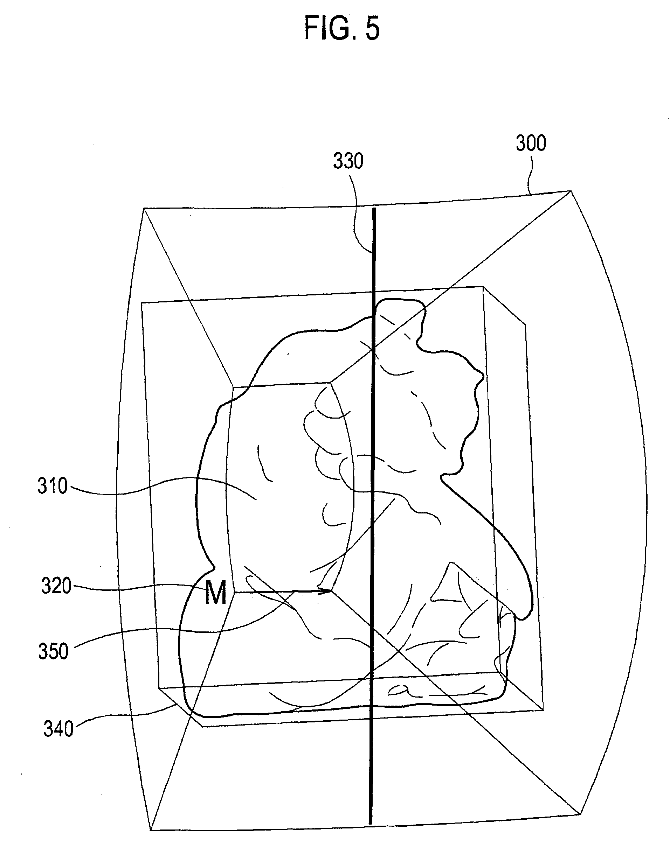

FIG. 5 is a schematic diagram showing an example of an orientation help view. Referring to FIG. 5, the OH view 300, which may show a spatial orientation of the volume data, includes the three-dimensional ultrasound image 310, a probe orientation marker 320 indicating a position of the ultrasound probe 112, a clip plane 330 indicating the images of the selected reference plane, a ROI 340 and a scanning direction marker 350.

In one embodiment, the probe orientation marker 320 may be formed based on the orientation of the ultrasound probe 112 outputted from the orientation detecting section of the ultrasound probe 112. Further, the scanning direction marker 350 may be formed based on the scanning direction outputted from orientation detection section of the ultrasound probe 112. The three-dimensional ultrasound image 310 may be overlapped with the OH view 300. In one embodiment, by way of non-limiting examples, the scanning direction marker 350 may include a first scanning direction marker indicating a swing direction (the elevation direction as illustrated in FIG. 3) of the array transducer and a second scanning direction marker indicating a scan line direction (the axial direction as illustrated in FIG. 3). The OH view forming section 133 may set the geometrical operation such as rotation, reduction, enlargement and movement of the OH view 300 in response to the third user instruction.

Referring back to FIG. 1, the display unit 140 may display the OH view 300, which is overlapped with the selected image of the reference planes and display the images of the A, B and C planes. The display unit 140 may further display on the screen the three-dimensional ultrasound image together with the images of the reference planes.

Any reference in this specification to "one embodiment," "an embodiment," "example embodiment," "illustrative embodiment," etc. means that a particular feature, structure or characteristic described in connection with the embodiment is included in at least one embodiment of the present invention. The appearances of such phrases in various places in the specification are not necessarily all referring to the same embodiment. Further, when a particular feature, structure or characteristic is described in connection with any embodiment, it is submitted that it is within the purview of one skilled in the art to affect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the spirit and scope of the principles of this disclosure. More particularly, numerous variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound system, comprising:
an ultrasound data acquisition unit (110) including an ultrasound probe for transmitting ultrasound signals to a target object and receiving ultrasound echo signals reflected from the target object, the ultrasound data acquisition unit (110) being configured to acquire ultrasound data based on the ultrasound echo signals, the ultrasound data acquisition unit (110) being further configured to detect an orientation and a scanning direction of the ultrasound probe (112); and
a processor (130) configured to form a volume data based on the ultrasound data, a three-dimensional ultrasound image (310) using the volume data, and an orientation help, OH , view showing a spatial orientation of the volume data on a display region, the OH view (300) including the three-dimensional ultrasound image (310), a probe orientation marker (320) indicating the detected orientation of the ultrasound probe (112), and a scanning direction marker (350) indicating the detected scanning direction, wherein the three-dimensional ultrasound image (310) is overlapped with the OH view (300),
**characterized by** comprising:
a user interface (120) for receiving a first user instruction for setting a geometrical operation including rotation, reduction, enlargement and movement of the OH view (300), wherein the processor (130) is further configured to set the geometrical operation of the OH view (300) in response to the first user instruction, to provide a clip plane (330) indicating an image of a selected one of reference planes on the OH view (300), and
wherein the OH view (300) further includes the clip plane (330) and a region of interest (340).

2. The ultrasound system of Claim 1, wherein the scanning direction marker (350) includes a first scanning direction marker (350) indicating a swing direction of an array transducer of the ultrasound probe (112) and a second scanning direction marker (350) indicating a scan line direction.

3. The ultrasound system of Claim 1, wherein the processor (130) comprises:
a volume data forming section (114) for forming the volume data based on the ultrasound data;
an ultrasound image forming section for forming a three-dimensional ultrasound image and images of reference planes of the three-dimensional ultrasound image; and an OH view forming section (133) for forming the OH view (300).

4. The ultrasound system of Claim 3,
wherein the user interface (120) further receives a second user instruction for setting a region of interest, ROI, of the three-dimensional ultrasound image and a third user instruction for selecting one of the reference planes.

5. A method of providing an orientation help, OH, view in an ultrasound system including an ultrasound probe (112), the method comprising:
a) obtaining ultrasound data by an ultrasound data acquisition unit (110) within the ultrasound system (100);
b) detecting an orientation and a scanning direction of the ultrasound probe (112) by the ultrasound data acquisition unit (110);
c) forming a volume data based on the ultrasound data by a processor (130) within the ultrasound system (100),
d) forming a three-dimensional ultrasound image (310) using the volume data by the processor (130); and
e) forming the OH view showing a spatial orientation of the volume data on a display region, the OH view (300) including the three-dimensional ultrasound image (310), a probe orientation marker (320) indicating the detected orientation of the ultrasound probe (112), and a scanning direction marker (350) indicating the detected scanning direction,
f) forming the three-dimensional ultrasound image (310) that is overlapped with the OH view (300),
**characterized by**:
g) receiving a first user instruction for setting a geometrical operation including rotation, reduction, enlargement and movement of the OH view (300),
h) setting the geometrical operation of the OH view (300) in response to the first user instruction, and
i) providing a clip plane (330) indicating an image of a selected one of reference planes on the OH view (300),
wherein the OH view (300) includes the clip plane (330) and a region of interest (340).

6. The method of Claim 5, wherein the scanning direction marker includes a first scanning direction marker indicating a swing direction of an array transducer of the ultrasound probe and a second scanning direction marker indicating a scan line direction.

7. The method of Claim 5, further comprising:
j) forming a three-dimensional ultrasound image and images of reference planes of the three-dimensional ultrasound image by the processor;
k) setting a region of interest, ROI, of the three-dimensional ultrasound image through user instruction by the user interface within the ultrasound system; and
l) selecting one of the reference planes through user instruction by the user interface.

8. A computer readable medium comprising instructions that, when executed by a processor (130) performs a method of providing orientation help, OH , view in an ultrasound system (100) including an ultrasound probe (112), cause the processor (130) to perform steps comprising:
a) obtaining ultrasound data;
b) detecting an orientation and a scanning direction of an ultrasound probe (112);
c) forming a volume data based on the ultrasound data;
d) forming the three-dimensional ultrasound image (310) using the volume data by the processor (130); and
e) forming the OH view showing a spatial orientation of the volume data on a display region, the OH view (300) including the three-dimensional ultrasound image (310), a probe orientation marker (320) indicating the detected orientation of the ultrasound probe (112), and a scanning direction marker (350) indicating the detected scanning direction,
f) forming the three-dimensional ultrasound image (310) that is overlapped with the OH view (300),
**characterized by**:
g) receiving a first user instruction for setting a geometrical operation including rotation, reduction, enlargement and movement of the OH view (300),
h) setting the geometrical operation of the OH view (300) in response to the first user instruction, and
i) providing a clip plane (330) indicating an image of a selected one of reference planes on the OH view (300),
wherein the OH view (300) includes the clip plane (330) and a region of interest (340).

9. The computer readable medium of Claim 8, wherein the seaming direction marker (350) includes a first scanning direction marker indicating a swing direction of an array transducer of the ultrasound probe (112) and a second scanning direction marker indicating a scan line direction.

10. The computer readable medium of Claim 9, further comprising:
j) forming a three-dimensional ultrasound image and images of reference planes of the three-dimensional ultrasound image;
k) setting a region of interest (ROI) of the three-dimensional ultrasound image; and
l) selecting one of the reference planes.

## Patentansprüche

1. Ultraschallsystem, welches Folgendes aufweist:
eine Ultraschalldatenerfassungseinheit (110), die eine Ultraschallsonde zum Übertragen von Ultraschallsignalen zu einem Zielobjekt und Empfangen von von dem Zielobjekt reflektierten Ultraschallechosignalen aufweist, wobei die Ultraschalldatenerfassungseinheit (110) dafür vorgesehen ist, Ultraschalldaten basierend auf den Ultraschallechosignalen zu erfassen, wobei die Ultraschalldatenerfassungseinheit (110) des Weiteren dafür vorgesehen ist, eine Ausrichtung und eine Scanrichtung der Ultraschallsonde (112) zu detektieren; und
einen Prozessor (130), der dafür vorgesehen ist, Volumendaten basierend auf den Ultraschalldaten, ein dreidimensionales Ultraschallbild (310), welches die Volumendaten verwendet, und eine Ausrichtungshilfsansicht, AH-Ansicht, welche eine räumliche Ausrichtung der Volumendaten auf einem Anzeigebereich anzeigt, zu erzeugen,
wobei die AH-Ansicht (300) das dreidimensionale Ultraschallbild (310), eine Sondenausrichtungsmarkierung (320), die die detektierte Ausrichtung der Ultraschallsonde (112) anzeigt, und eine Scanrichtungsrichtungsmarkierung (350),
die die detektierte Scanrichtung anzeigt, aufweist,
wobei das dreidimensionale Ultraschallbild (310) mit der AH-Ansicht (300) überlappt,
**gekennzeichnet durch :**
eine Benutzerschnittstelle (120) zum Empfangen einer ersten Benutzereingabe zum Festlegen einer geometrischen Operation einschließlich Rotation, Verkleinerung, Vergrößerung und Bewegung der AH-Ansicht (300), wobei der Prozessor (130) des Weiteren dafür vorgesehen ist, die geometrische Operation der AH-Ansicht als Reaktion auf die erste Benutzereingabe festzulegen, eine Ausschnittsebene (330) zu erzeugen, welche eine Abbildung einer ausgewählten von Referenzebenen auf der AH-Ansicht (300) anzeigt, und wobei die AH-Ansicht (300) des Weiteren die Ausschnittsebene (330) und einen interessierenden Bereich (340) beinhaltet.

2. Ultraschallsystem nach Anspruch 1, wobei die Scanrichtungsmarkierung (350) eine erste Scanrichtungsmarkierung (350), die eine Schwingungsrichtung einer Matrixprüfsonde der Ultraschallsonde (112) anzeigt, und eine zweite Scanrichtungsmarkierung (350) aufweist, die eine Scanlinienrichtung anzeigt.

3. Ultraschallsystem nach Anspruch 1, wobei der Prozessor (130) Folgendes aufweist:
einen Volumendatenabschnitt (114) zum Erzeugen der Volumendaten basierend auf den Ultraschalldaten;
einen Ultraschallbilderzeugungsabschnitt zum Erzeugen eines dreidimensionalen Ultraschallbilds und Abbildungen von Referenzebenen des dreidimensionalen Ultraschallbilds; und
einen AH-Ansicht-Erzeugungsabschnitt (133) zum Erzeugen der AH-Ansicht (300).

4. Ultraschallsystem nach Anspruch 3, wobei die Benutzerschnittstelle (120) des Weiteren eine zweite Benutzereingabe zum Festlegen eines interessierenden Bereichs, ROI, des dreidimensionalen Ultraschallbilds und eine dritte Benutzereingabe zum Auswählen einer der Referenzebenen empfängt.

5. Verfahren zum Erzeugen einer Ausrichtungshilfs-, AH-Ansicht in einem Ultraschallsystem, welches eine Ultraschallsonde (112) aufweist, wobei das Verfahren Folgendes aufweist:
a) Erfassen von Ultraschalldaten mittels einer Ultraschalldatenerfassungseinheit (110) innerhalb des Ultraschallsystems (100);
b) Detektieren einer Ausrichtung und einer Scanrichtung der Ultraschallsonde (112) durch die Ultraschalldatenerfassungseinheit (110);
c) Erzeugen von Volumendaten, basierend auf den Ultraschalldaten, mittels eines Prozessors (130) innerhalb des Ultraschallsystems (100);
d) Erzeugen eines dreidimensionalen Ultraschallbilds (310) unter Verwendung der Volumendaten mittels des Prozessors (130); und
e) Erzeugen der AH-Ansicht, welche eine räumliche Ausrichtung der Volumendaten auf einem Anzeigebereich zeigt, wobei die AH-Ansicht (300) das dreidimensionale Ultraschallbild (310), eine Sondenausrichtungsmarkierung (320), welche die detektierte Ausrichtung der Ultraschallsonde (112) anzeigt, und eine Scanrichtungsmarkierung (350), welche die detektierte Scanrichtung anzeigt, aufweist;
f) Erzeugen des dreidimensionalen Ultraschallbilds (310), welches mit der AH-Ansicht (300) überlappt,
**gekennzeichnet durch :**
g) Empfangen einer ersten Benutzereingabe zum Festlegen einer geometrischen Operation einschließlich Rotation, Verkleinerung, Vergrößerung und Bewegung der AH-Ansicht (300),
h) Festlegen der geometrischen Operation der AH-Ansicht (300) als Reaktion auf die erste Benutzereingabe, und
i) Erzeugen einer Ausschnittsebene (330), welche eine Abbildung einer ausgewählten von Referenzebenen auf der AH-Ansicht (300) anzeigt, wobei die AH-Ansicht (300) die Ausschnittsebene (330) und einen interessierenden Bereich (340) aufweist.

6. Verfahren nach Anspruch 5, wobei die Scanrichtungsmarkierung eine erste Scanrichtungsmarkierung, die eine Schwingungsrichtung einer Matrixprüfsonde der Ultraschallsonde anzeigt, und eine zweite Scanrichtungsmarkierung aufweist, die eine Scanlinienrichtung anzeigt.

7. Verfahren nach Anspruch 5, welches des Weiteren Folgendes aufweist:
j) Erzeugen eines dreidimensionalen Ultraschallbilds und Abbildungen von Referenzebenen des dreidimensionalen Ultraschallbilds mittels des Prozessors;
k) Festlegen eines interessierenden Bereichs, ROI, des dreidimensionalen Ultraschallbilds durch eine Benutzereingabe durch die Benutzerschnittstelle innerhalb des Ultraschallsystems; und
l) Auswählen einer der Referenzebenen durch eine Benutzereingabe mittels der Benutzerschnittstelle.

8. Computerlesbares Medium, welches Befehle aufweist, die, wenn sie von einem Prozessor (130) ausgeführt werden, ein Verfahren zum Erzeugen einer Ausrichtungshilfs-, AH-Ansicht in einem Ultraschallsystem (100), welches eine Ultraschallsonde (112) aufweist, durchführt, wobei bewirkt wird, dass der Prozessor (130) Schritte ausführt, welche Folgendes aufweisen:
a) Erfassen von Ultraschalldaten;
b) Detektieren einer Ausrichtung und einer Scanrichtung einer Ultraschallsonde (112);
c) Erzeugen von Volumendaten basierend auf den Ultraschalldaten;
d) Erzeugen des dreidimensionalen Ultraschallbilds (310) unter Verwendung der Volumendaten mittels des Prozessors (130); und
e) Erzeugen der AH-Ansicht, welche eine räumliche Ausrichtung der Volumendaten auf einem Anzeigebereich zeigt, wobei die AH-Ansicht (300) das dreidimensionale Ultraschallbild (310), eine Sondenausrichtungsmarkierung (320), welche die detektierte Ausrichtung der Ultraschallsonde (112) anzeigt, und eine Scanrichtungsmarkierung (350), welche die detektierte Scanrichtung anzeigt, aufweist;
f) Erzeugen des dreidimensionalen Ultraschall bilds (310), welches mit der AH-Ansicht (300) überlappt,
**gekennzeichnet durch :**
g) Empfangen einer ersten Benutzereingabe zum Festlegen einer geometrischen Operation einschließlich Rotation, Verkleinerung, Vergrößerung und Bewegung der AH-Ansicht (300),
h) Festlegen der geometrischen Operation der AHA-Ansicht (300) als Reaktion auf die erste Benutzereingabe, und
i) Erzeugen einer Ausschnittsebene (330), welche eine Abbildung einer ausgewählten von Referenzebenen auf der AH-Ansicht (300) anzeigt, wobei die AH-Ansicht (300) die Ausschnittsebene (330) und einen interessierenden Bereich (340) aufweist.

9. Computerlesbares Medium nach Anspruch 8, wobei die Scanrichtungsmarkierung (350) eine erste Scanrichtungsmarkierung (350), die eine Schwingungsrichtung einer Matrixprüfsonde der Ultraschallsonde (112) anzeigt, und eine zweite Scanrichtungsmarkierung (350) aufweist, die eine Scanlinienrichtung anzeigt.

10. Computerlesbares Medium nach Anspruch 9, welches des Weiteren Folgendes aufweist:
j) Erzeugen eines dreidimensionalen Ultraschallbilds und Abbildungen von Referenzebenen des dreidimensionalen Ultraschallbilds;
k) Festlegen eines interessierenden Bereichs (ROI) des dreidimensionalen Ultraschallbilds; und
l) Auswählen einer der Referenzebenen.

## Revendications

1. Système ultrasonique comportant :
une unité d'acquisition de données ultrasoniques (110) comprenant une sonde ultrasonique pour transmettre des signaux ultrasoniques sur un objet cible et recevoir des signaux d'écho ultrasoniques réfléchis par l'objet cible, l'unité d'acquisition de données ultrasoniques (110) étant configurée pour acquérir des données basées sur les signaux d'écho ultrasoniques, l'unité d'acquisition de données ultrasoniques (110) étant en outre agencée pour détecter une orientation et une direction de balayage de la sonde ultrasonique (112) ; et
un processeur (130) agencé pour constituer une donnée de volume basée sur les données ultrasoniques, une image ultrasonique tridimensionnelle (310) utilisant la donnée de volume et une assistance d'orientation, OH, une vue représentant une orientation spatiale des données de volume dans une zone d'affichage, la vue OH (300) incluant l'image ultrasonique tridimensionnelle (310), un marqueur (320) d'orientation de la sonde indiquant l'orientation détectée de la sonde ultrasonique (112), et un marqueur de direction de balayage (350) indiquant la direction de balayage détectée, dans lequel l'image ultrasonique tridimensionnelle (310) est recouverte par la vue OH (300),
**caractérisé en ce qu'**il comprend :
un interface utilisateur (120) pour recevoir un première instruction d'utilisateur pour effectuer une opération géométrique comportant la rotation, l'agrandissement et le mouvement sur la vue OH (300), dans lequel le processeur (130) est en outre configuré pour effectuer l'opération géométrique de la vue OH (300) en réponse aux premières instructions d'utilisateur, pour fournir un plan de vue (330) montrant une image d'un des plans de référence de la vue OH (300), et dans lequel la vue OH (300) comprend en outre le plan de vue (330) et une zone d'intérêt (340).

2. Système ultrasonique selon la revendication 1, dans lequel le marqueur de direction de balayage (350) comprend un premier marqueur de direction de balayage (350) indiquant une direction de déviation d'un transducteur de tableau d'une sonde ultrasonique (112) et un second marqueur de direction de balayage (350) indiquant une ligne de direction de balayage.

3. Système ultrasonique selon la revendication 1, dans lequel le processeur (130) comporte :
une section (114) de création de données de volume pour créer des données de volume basées sur les données ultrasoniques ;
une section de création d'image ultrasonique pour créer une image ultrasonique tridimensionnelle et des images de plans de référence de l'image ultrasonique tridimensionnelle; et une section (133) de création d'une vue OH pour créer une vue OH (300).

4. Système ultrasonique selon la revendication 3,
dans lequel l'interface utilisateur (120) reçoit en outre une seconde instruction d'utilisateur pour fixer une zone d'intérêt, ROI, de l'image ultrasonique tridimensionnelle; et une troisième instruction d'utilisateur pour sélectionner un des plans de référence.

5. Procédé pour effectuer une aide d'orientation, OH vue dans un système ultrasonique comportant une sonde ultrasonique (112), le procédé comportant :
a) l'obtention de données ultrasoniques par une unité d'acquisition de données ultrasoniques (110) dans le système ultrasonique (100) ;
b) la détection d'une orientation et d'une orientation de balayage de la sonde ultrasonique (112) par l'unité d'acquisition de données ultrasoniques (110) ;
c) la création de données de volume basées sur les données ultrasoniques par un processeur (130) dans le système ultrasonique (100) ;
d) la création d'une image ultrasonique tridimensionnelle (310) utilisant les données de volume par le processeur (130) : et
e) la formation de la vue OH montrant une orientation spatiale des données de volume dans une zone d'affichage, de la vue OH (300) incluant l'image tridimensionnelle ultrasonique (310), un marqueur de sonde d'orientation (320) indiquant l'orientation détectée de la sonde ultrasonique (112) et un marqueur (350) de direction de balayage indiquant la direction détectée du balayage,
f) la création de l'image ultrasonique tridimensionnelle (310) qui est recouverte par la vue OH (300),
**caractérisé par** :
g) la réception d'une première instruction d'utilisateur pour effectuer une opération géométrique comportant la rotation, l'agrandissement, et le mouvement sur la vue OH (300),
h) la réalisation de l'opération géométrique de la vue OH (300) en réponse aux premières instructions d'utilisateur, et
i) la fourniture d'un plan de vue (330) montrant une image sélectionnée d'un des plans de référence de la vue OH (300),
dans lequel la vue OH (300) comprend en outre le plan de vue (330) et une zone d'intérêt (340).

6. Procédé selon la revendication 5, dans lequel le marqueur de direction de balayage inclut un premier marqueur de direction de balayage indiquant la direction de déviation d'un transducteur de tableau d'une sonde ultrasonique et un second marqueur de direction de balayage indiquant une ligne de direction de balayage.

7. Procédé selon la revendication 5, comprenant en outre :
j) la création d'une image ultrasonique tridimensionnelle et des images de plans de référence de l'image ultrasonique tridimensionnelle par le processeur ;
k) la détermination d'une zone d'intérêt, ROI, de l'image ultrasonique tridimensionnelle au travers de l'instruction d'utilisateur par l'interface d'utilisateur dans le système ultrasonique ; et
l) la sélection d'un des plans de référence au travers de l'instruction d'utilisateur par l'interface d'utilisateur.

8. Support de programme lisible par ordinateur, comportant des instructions qui, lorsqu'il est mis en oeuvre par un processeur (130) exécute un procédé d'assistance à l'orientation, OH, vue dans un système ultrasonique (100) comportant une sonde ultrasonique (112), impose au processeur (130) d'exécuter les étapes comprenant :
a) l'obtention de données ultrasoniques ;
b) la détection d'une orientation et d'une direction de balayage de la sonde ultrasonique (112) ;
c) la création d'une donnée de volume basée sur la donnée ultrasonique ;
d) la création d'une image ultrasonique tridimensionnelle (310) utilisant la donnée de volume par le processeur (130) ; et
e) la formation d'une vue OH montrant une orientation spatiale de la donnée de volume sur une zone d'affichage, la vue OH (300) incluant l'image ultrasonique tridimensionnelle (310), un marqueur (320) d'orientation de la sonde indiquant l'orientation détectée de la sonde ultrasonique (112), et un marqueur de direction de balayage (350) indiquant la direction de balayage détectée,
f) la formation de l'image ultrasonique tridimensionnelle (310) qui est recouverte par la vue OH (300),
**caractérisé par** :
g) la réception d'une première instruction d'utilisateur pour fixer une opération géométrique comportant la rotation, l'agrandissement, et le mouvement sur la vue OH (300),
h) la réalisation de l'opération géométrique de la vue OH (300) en réponse aux premières instructions d'utilisateur, et
i) la fourniture d'un plan de vue (330) montrant une image d'un des plans de référence de la vue OH (300),
dans lequel la vue OH (300) inclut le plan de vue (330) et une zone d'intérêt (340).

9. Support de programme lisible par ordinateur selon la revendication 8, dans lequel le marqueur de direction de balayage (350) inclut un premier marqueur de direction de balayage indiquent la direction de déviation d'un transducteur de tableau d'une sonde ultrasonique (112) et un second marqueur de direction de balayage indiquant une ligne de direction de balayage.

10. Support de programme lisible par ordinateur selon la revendication 9, comprenant en plus :
j) la formation d'une image ultrasonique tridimensionnelle et d'images de plans de référence de l'image ultrasonique tridimensionnelle ;
k) la détermination d'une zone d'intérêt, ROI, de l'image ultrasonique tridimensionnelle ; et
l) la sélection d'un des plans de référence.
